# EUROPEAN PATENT APPLICATION

(11) **EP 1 085 088 A1**
(43) Date of publication of application: **21.03.2001**
(21) Application number: 99922492.6
(22) Date of filing: 26.05.1999
(51) Int. Cl.: C12N 15/08, C12P 21/08, C12N 5/20, C07K 16/36, G01N 33/53, G01N 33/577

(54) **MONOCLONAL ANTIBODIES AND METHOD FOR ASSAYING FDP**

(30) Priority: 02.06.1998 JP 15274398
(71) Applicant: TEIKOKU HORMONE MFG. CO., LTD., Tokyo 107-8522 (JP)
(72) Inventor: TANABE, Hiroyuki, Kawasaki-shi, Kanagawa 214-0004 (JP); YAMAZAKI, Kazushige, Ebina-shi, Kanagawa 234-0406 (JP); NONAKA, Kazuhiko, Hino-shi, Tokyo 191-0062 (JP); TSUCHIYA, Wataru, Kawasaki-shi, Kanagawa 211-0053 (JP); OKUMURA, Hayato, Kawasaki-shi, Kanagawa 214-0004 (JP); OHOISHI, Tatsuo, Yokohama-shi, Kanagawa 225-0023 (JP); MANITA, Hideaki, Sagamihara-shi, Kanagawa 228-0817 (JP)
(74) Representative: Weisert, Annekäte, Dipl.-Ing. Dr.-Ing.
(86) International application number: JP9902764
(87) International publication number: WO9963075

(57) **Abstract**

The present invention provides two kinds of monoclonal antibodies which do not react with specific fractions in human fibrin/fibrinogen degradation products by plasmin and discloses a method of measuring FDP by using plasma as a specimen using the two kinds of the monoclonal antibodies according to the immunological agglutination method.

## Description

### Technical Field

This invention relates to novel two kinds of monoclonal antibodies, and a method for assaying human fibrin/fibrinogen degradation products by plasmin (hereinafter referred to as "FDP") in blood plasma using the same.

### Background art

Blood does not form coagulation in physiological conditions since it is flowing in a blood vessel. However, when a wall of the blood vessel is injured by an external injury or arteriosclerosis, etc., fluidity of the blood is lost and platelets are adhered to the portion to be injured to start coagulation reaction. Also, in blood plasma, a coagulation factor is successively activated to appear thrombin. This thrombin acts on fibrinogen to cut part of the molecule whereby converting it into a fibrin monomer and the fibrin monomers are polymerized to form a thrombus. On the other hand, the fibrin in the thrombus formed in the blood vessel is dissolved by accepting an action of plasmin which is a protease to appear a decomposed product of fibrin in flowing blood (fibrinolysis). Also, the fibrinogen in blood plasma is also decomposed (fibrinogenolysis) by accepting an action of plasmin when plasmin an activity of which is not inhibited by an anti-plasmin substance is present. And several kinds of the decomposed products produced by these fibrinogenolysis and fibrinolysis, i.e., the presence of FDP suggests that a thrombus is formed in a blood vessel whereby dissolution thereof is occurred, and/or plasmin in the blood plasma is activated by lowering in fibrinogenolysis inhibiting factor, etc. and an anti-plasmin activity is exceeded.

Accordingly, measurement of FDP in blood has now widely been used in diagnoses of disseminated intravascular coagulation (DIC), diseases based on thrombus and circulation disorder, diseases which cause a hemorrhage tendency, diseases remarkable in fibrinogenolysis activity sthenia, etc.

In an immunological assay method of FDP, immunological coagulation reaction using a latex reagent in which an anti-human fibrinogen polyclonal antibody is sensitized to latex particles has heretofore been generally used. This method can measure whole kinds of FDP (Total FDP). However, when a blood in which fibrinogen is not removed, i.e., blood plasma is used as a specimen, it becomes suspectedly positive by the fibrinogen even when no FDP is present. Thus, a serum from which fibrinogen is removed must be used as a specimen.

In order to solve the above-mentioned problems, some monoclonal antibodies having properties not reacting with fibrinogen have been proposed (see Japanese Patent Publications (Kokoku) No. 83240/1993, No. 61639/1992 and No. 48119/1993), and some assay methods of FDP using these monoclonal antibodies have also been proposed (see Japanese Patent Publications (Kokoku) No. 38906/1993, No. 38907/1993 and No. 46104/1995). However, according to the method using these monoclonal antibodies, it is possible to specifically assay FDP at a specific fraction but total FDP cannot be assayed.

On the other hand, when a minute component in a living sample is to be measured, it has widely spread a method of simultaneously and automatically measuring several kinds of items to be inspected by using a device in recent years. As a specimen in the inspection of blood coagulation type factors, blood plasma has been used in almost all the cases. Accordingly, it has earnestly been desired to develop an assay method using blood plasma as a specimen in the measurement of total FDP.

The present inventors have succeeded in obtaining two kinds of novel monoclonal antibodies having properties of not reacting with fibrinogen by using FDP prepared from whole blood as an antigen. And according to an immunological agglutination reaction using these two kinds of monoclonal antibodies, they have found that total FDP can be measured with good correlation with the case of using serum as a specimen, even when blood plasma is used as a specimen.

### Disclosure of the invention

According to the present invention, two kinds of novel monoclonal antibodies wherein
(1) a monoclonal antibody having properties of specifically reacting with X fraction and Y fraction, but not reacting with D fraction, E fraction, DD fraction and Fg, in plasmin degradation products of human fibrinogen (hereinafter referred to as "Fg") or human fibrin and
(2) a monoclonal antibody having properties of reacting with X fraction, Y fraction, D fraction, E fraction and DD fraction, but not reacting with Fg can be provided.

Also, according to the present invention, a method of assaying FDP by an immunological agglutination reaction with the use of these two kinds of monoclonal antibodies having properties of (1) and (2) as mentioned above as an antibody.

### Brief description of the drawings

Fig. 1 is a graph showing a relationship between the reaction rate and concentrations of the respective fractions in FDP;
Fig. 2 is a graph showing a correlation between the FDP concentration in serum of the reagent according to the present invention and the FDP concentration in blood plasma specimen; and
Fig. 3 is a graph showing a correlation between the FDP concentration according to the reagent of the conventional method and the FDP concentration according to the reagent of the present invention.

### Best mode for carrying out the invention

For producing the novel monoclonal antibody of the present invention, FDP prepared from whole blood is used as an antigen. Said FDP can be generally prepared by coagulating human whole blood with thrombin, acting plasmin thereon to dissolve the material to make a digested material of the coagulation mass, and the digested material is adsorbed in an insoluble carrier to which an anti-Fg antibody and anti-FDP antibody as ligands, and then, by making dissolved out.

The monoclonal antibody of the present invention can be obtained, for example, according to the general method of Köhler and Milstein (Nature, Vol. 256, pp. 495-497 (1975)), by fusing a spleen cell and a myeloma cell of mouse immunized by the FDP prepared as mentioned above, selecting cells which produce the aimed antibody from these fused cells by the ELISA method which has generally been known as a general method of an inspection method of an antibody, and culturing the selected fused cell.

The immunological coagulation reaction using two kinds of the monoclonal antibodies according to the present invention can be carried out by, for example, preparing a latex reagent to which the monoclonal antibody having the above-mentioned characteristics of (1) is sensitized and a latex reagent to which the monoclonal antibody having the above-mentioned characteristics of (2) is sensitized, reacting a latex reagent in which the above two kinds of latex reagents are mixed with a specimen, and measuring the degree of coagulation by a spectroscopic method or with eyes. Also, the immunological coagulation reaction can be carried out by using, as a latex reagent, one kind of a latex reagent in which a mixture of the monoclonal antibody having the above-mentioned characteristics of (1) and the monoclonal antibody having the above-mentioned characteristics of (2) is sensitized to latex particles, and reacting the latex reagent with a specimen. The latex particles as a carrier are preferably polystyrene latex particles, and the particle size is suitably 0.05 to 2.0 µm or so in general. Also, as a method for sensitizing the monoclonal antibody to the latex particles, a method known in the art, for example, either of the methods in which a method of physically adsorbing the antibody to the latex particles or a method of chemically binding, may be used.

In the present invention, when measurement is carried out by a spectroscopic method, it can be carried out by mixing a predetermined amount of the latex reagent and a specimen, and spectroscopically measuring an increase in absorbance after a predetermined period of time at a suitable wavelength. Also, when it is measured with eyes, a predetermined amount of the latex reagent and a specimen are dropped on a slide plate or a microtiter plate and after mixing a predetermined period of time, presence or absence of agglutination is observed with eyes. In particular, the spectroscopic method is a preferred method since several kinds of items to be inspected can be simultaneously and automatically measured when blood plasma is used as a specimen.

According to the above, even when blood plasma is used as a specimen, measurement of a total FDP can be realized with a good correlation as in the case where serum is used as a specimen.

### EXAMPLES

In the following, the present invention will be explained in more detail by referring to Examples.

### Example 1

### Production of FDP antigen for immune

Preparation of a FDP antigen for immune was carried out accroding to the method of P.W. Koppert et al. (Blood, 68, 437-441, 1986). Normal whole blood obtained by venipuncture was apportioned each 10 ml in two plastic tubes into which 900 IU of tissue type plasminogen activator (available from Sigma Co.) and immediately mixed. Next, this blood was transferred to a centrifugal tube made of glass into which 10 NIHU of respective thrombin (available from NACALAI TESQUE INC.) and coagulated, and then, incubated at 37°C. Incubation was carried out until the coagulated mass were completely dissolved (45 minutes), and the reaction was terminated by adding 1,000 KIU of Aprotinin (trade name, available from Okura Seiyaku K.K.) and 10 mM of diisopropyl fluorophosphate (available from Sigma Co.) to one of the centrifugal tube. With regard to the remaining centrifugal tube, incubation was further continued for 120 minutes and the reaction was terminated in the same manner. After stopping the reaction, the respective centrifugal tubes were centrifuged at 2300 g for 20 minutes, and supernatants were separated to obtain two kinds of whole blood coagulated mass digested products BCL-1 (incubation time: 45 minutes) and BCL-2 (incubation time: 165 minutes) different in incubation time each in an amount of 5 ml.

The material in which 1 ml of the above-mentioned whole blood coagulated mass digested product diluted 4 times with a borate buffer was added to anti-FDP/CH Sepharose 4B column (gel content: 4 ml) prepared by binding anti-Fg, anti-D, anti-E, anti-FgDP and anti-FbDP antibodies to activated CH Sepharose 4B (trade name, available from Pharmacia Co.). After successively washing it with 100 ml of a borate buffer, 20 ml of a borate buffer containing 0.5M of NaCl and 20 ml of a borate buffer diluted 10-times, a fraction adsorbed to the column was eluted with a 0.01M NaOH solution. The eluted fraction was rapidly neutralized with a 0.1M HCl solution and dialyzed with purified water, and then, lyophilized to obtain 1.2 mg of FDP-1 and 1.0 mg of FDP-2 as FDP antigens for immune from each 5 ml of BCL-1 and BCL-2, respectively.

### Example 2

### 2-a) Production of anti-FDP monoclonal antibody

To BALB/C mouse (6 to 8 weeks-old) were subcutaneously injected 30 µg of FDP-1 antigen produced in Example 1 with a Freund's complete adjuvant three times with each three weeks intervals, and finally 60 µg thereof was intravenously injected.

After 3 days from the final immunization, spleen of the mouse was delivered to collect spleen cells, and after washing with Delbecco's Modified minimum basic medium (hereinafter referred to as "D MEM") three times. Thereafter, a number of cells were counted, and its 2.8 x 10⁸ cells were mixed with 5.6 x 10⁷ cells of mouse myeloma cell P₃-NS-1/1-Ag4-1 (hereinafter referred to as "NS-1") and the mixture was centrifuged to collect cells. To the pellet was added 1 ml of a polyethylene glycol solution (D MEM containing PEG-100: 4.25%, DMSO: 1.5%) warmed to 37°C, and the centrifugal tube was slowly rotated for 1 minute to fuse the cells. D MDM having a temperature of 37°C was added to the fused cells in an amount of each 2 ml 10 times in total with each 30 seconds intervals, and then, the cells were centrifuged. The pellet obtained was suspended in RPMI-1640 medium containing 20% fetal bovine serum so that a number of spleen cells became 4 x 10⁵ cells/ml, and the suspension was apportioned to 96-well microplate each in an amount of 0.1 ml and cultured in a 5% CO₂-containing incubator. After 24 hours, a half amount of the supernatant of each well was disposed, 0.1 ml of a HAT medium (hypoxanthine-aminopterin-thymidine, RPMI-1640 medium containing 10% fetal bovine serum) was added to the residue. Thereafter, these cells were cultivated for 2 weeks while a half amount of the HAT medium was exchanged with each 3 or 4 days, and an antibody activity of the cultivated supernatant in the grown well was measured by the ELISA method of the following 2-b). As a result, with regard to the cultivated supernatant in which a FDP antibody production was admitted, specificity of the antibody was confirmed by the ELISA method of the following 2-c).

The cells of wells in which the activity was admitted were diluted by RPMI-1640 medium containing 10% fetal bovine serum which contains BALB/C mouse thymocytes, and subjected to cloning by the limiting dilution method to obtain a monoclonal antibody (FDP1-1H10)-producing fused cell which specifically reacts with X fraction and Y fraction but does not react with Fg, D fraction, E fraction and DD fraction. Cells with a number of 1 x 10⁷ cells or more were intraperitoneally administered to a BALB/C mouse to which 0.5 ml of pristane was previously administered to make an ascites tumor whereby ascites was obtained. This ascites was purified by Prosep-A column (trade name, available from Bioprocessing Co.), and lyophilized to obtain an anti-FDP monoclonal antibody (FDP1-1H10) as a white powder.

With regard to the FDP-2 antigen, immune to mouse (subcutaneously administered, 4 times with 3-weeks intervals) and cell fusion were carried out similarly to obtain a monoclonal antibody (FDP2-4B3)-producing fused cell which reacts with X fraction, Y fraction, D fraction, E fraction and DD fraction but does not react with Fg by cloning. Then, ascites was prepared and the ascites was purified to obtain an anti-FDP monoclonal antibody (FDP2-4B3).

### 2-b) Screening method of anti-FDP monoclonal antibody

Screening of the anti-FDP antibody activity in the culture supernatant was carried out by the ELISA method. Rabbit anti-mouse IgG was immobilized to 96-well immunoplate (C96 MAXISORP, trade name, available from Nunc Co.), and then, 25 µl of culture supernatant was added to the respective wells, and allowed to stand at room temperature for one hour. Thereafter, the respective wells were washed with a 10 mM phosphate buffer containing 0.05% of Tween 20 (pH 7.4) three times, and 100 µl of 5 µg/ml Fg or FDP (mixed antibodies of DD, X and Y) prepared by the same buffer solution was added to the respective wells and reacted at room temperature for 30 minutes. After washing three times with the same buffer solution, 100 µl of a peroxidase-labeled rabbit anti-Fg antibody was added to the respective wells and reacted at room temperature for 30 minutes. Next, after washing three times with the same buffer solution, 100 µl of a substrate solution containing 0.13% 2,2'-azinobis(3-ethylbenzothiazolin-6-sulfonic acid) diammonium prepared by 0.1 M citrate buffer solution (pH 4.2) and 0.007% hydrogen peroxide was added to the respective wells and allowed to stand at room temperature for 30 minutes. Then, 100 µl of 2 mM sodium azide was added to the respective wells to terminate the reaction and absorbances of the respective wells at 405 nm were measured. According to this ELISA method, a hybridoma which secretes an antibody which does not react with Fg and reacts with FDP, i.e., an anti-FDP antibody-producing fused cell was selected.

### 2-c) Specificity of anti-FDP monoclonal antibody

Specificity of the anti-FDP monoclonal antibody was confirmed by the ELISA method which is the same as used in the above-mentioned 2-b). That is, to a rabbit anti-mouse IgG-fixed plate was added 25 µl of a diluted row (1 to 10⁷-fold) of the culture supernatants, and x, Y, DD, D and E fractions of FDP were separately added to measure the reactivity to the respective fractions. Incidentally, when the reactivity to the E fraction is to be measured, a peroxidase-labeled rabbit anti-E antibody was used in place of the peroxidase-labeled rabbit anti-Fg antibody.

Specificities of the anti-FDP monoclonal antibodies (FDP1-1H10) and (FDP2-4B3) produced in the above-mentioned 2-a) were as shown in Table 1.

**Table 1**

| | FDP1-1H10 | FDP2-4B3 |
|---|---|---|
| X | + | + |
| Y | + | + |
| DD | - | + |
| D | - | + |
| E | - | + |
| Fg | - | - |

### Example 3

### Production of anti-FDP monoclonal antibody-sensitized latex reagent

### 3-a) Reagent for spectroscopic measurement

The anti-FDP monoclonal antibodies (FDP1-1H10) and (FDP2-4B3) produced in the above-mentioned 2-a) were each prepared so that they became 1 mg/ml with a glycine buffer solution (0.23 M glycine, 0.9% NaCl and 0.1% NaN₃). These antibody solutions and 10% polystyrene latex (available from Nippon Synthetic Rubber K.K.) suspension having an average particle diameter of 0.181 µm were mixed with a ratio of 10:1 and the mixture was stirred at room temperature for one hour to sensitize the antibodies to the polystyrene latex particles. Unreacted monoclonal antibodies which were not adsorbed to the polystyrene latex were removed by centrifugation (20,000 x g, for 30 minutes), and the residue was suspended in a Tris buffer solution (0.1M Tris-HCl buffer solution) containing 0.4% BSA so that the suspended amount became 1%. After stirring at room temperature for 30 minutes, unreacted BSA solution was removed by centrifugation (20,000 x g, for 30 minutes), and the residue was made a 0.33% suspension. These two kinds of 0.33% suspensions were mixed in an amount of 1:1 to produce a reagent for spectroscopic measurement.

### 3-b) Reagent for slide

In the same manner as in the above-mentioned 3-a), 1.0% suspensions of the anti-FDP monoclonal antibodies (FDP1-1H10) and (FDP2-4B3)-sensitized polystyrene latex particles were each prepared and mixed with a ratio of 1:1 to produce a reagent for slide.

### Example 4

### Reactivity with the respective FDP fractions in FDP

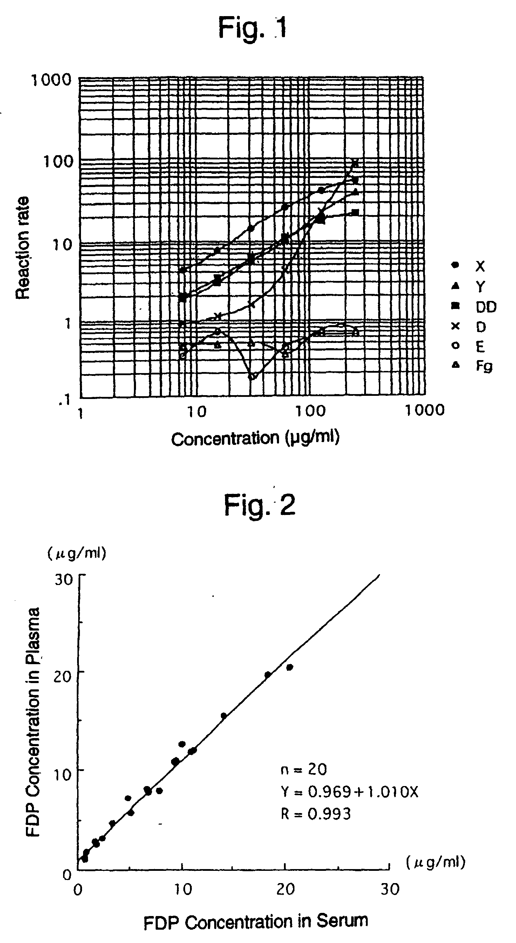

Reactivities with X fraction, Y fraction, DD fraction, D fraction, E fraction and Fg were examined by using LPIA100 (trade name, available from Mitsubishi Chemical Co., Ltd.). To a reaction cell containing the respective FDP fraction solutions (10 µl) and 200 µl of Tris buffer was injected 40 µl of the anti-FDP monoclonal antibody-sensitized latex reagent produced in 3-a) as mentioned above, and an absorbance at 950 nm was measured for 10 minutes to calculate the change in absorbance. The results are shown in Fig. 1. From Fig. 1, it showed reactivities with the X fraction, Y fraction, DD fraction and D fraction, but did not show reactivities with the E fraction and Fg.

### Example 5

### Correlation of the measured values between plasma and serum specimen

By using LPIA100 (trade name, available from Mitsubishi Chemical Co., Ltd.), patient plasma and serum prepared by removing fibrin from patient plasma using a FDP-exclusive blood collecting tube (available from Teikoku Hormone MFG Co., Ltd.) were measured by using the anti-FDP monoclonal antibody-sensitized latex reagent produced by 3-a) as mentioned above, and correlation of the measured values of FDP between the plasma and serum specimen was examined. As the standard product, a FDP prepared from whole blood or plasma was used. To a reaction cell containing 5 µl of a specimen and 200 µl of a Tris buffer solution (pH 8.2) was injected 40 µl of the anti-FDP monoclonal antibody-sensitized latex reagent, and then, absorbance at 950 nm was measured for 10 minutes, and a FDP concentration in the specimen was determined from the change in absorbance. The results are shown in Fig. 2. From Fig. 2, it showed extremely good correlation (correlation coefficient: 0.993), and the measured value substantially accorded with the regression formula: y (plasma) = 0.969 + 1.010X (serum) (n=20).

### Example 6

### Correlation with anti-Fg polyclonal antibody-sensitized latex reagent

### 6-a) Spectroscopic measurement

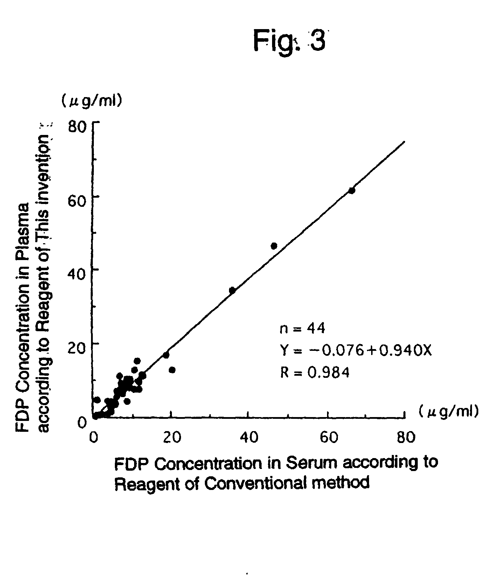

By using LPIA100 (trade name, available from Mitsubishi Chemical Co., Ltd.), patient plasma and serum prepared by removing fibrin from patient plasma using a FDP-exclusive blood collecting tube (available from Teikoku Hormone MFG Co., Ltd.) were measured by using the anti-FDP monoclonal antibody-sensitized latex reagent produced by 3-a) as mentioned above and an anti-Fg polyclonal antibody-sensitized latex reagent (trade name: "ELPIA·FDP", available from Teikoku Hormone MFG Co., Ltd.) of the conventional method, and correlation of the measured values of FDP between the reagent of the present invention and the reagent of the conventional method was examined. As the standard product, a FDP prepared from whole blood or plasma was used. To a reaction cell containing 5 µl of a specimen and 200 µl of a Tris buffer solution (pH 8.2) was injected 40 µl of the anti-FDP monoclonal antibody-sensitized latex reagent, and then, absorbance at 950 nm was measured for 10 minutes, and a FDP concentration in the specimen was determined from the change in absorbance. The results are shown in Fig. 3. From Fig. 3, it showed extremely good correlation (correlation coefficient: 0.984), and the measured value substantially accorded with the regression formula: y (present method) = -0.076 + 0.940X (conventional method) (n=44).

### 6-b) Slide agglutination method

Respective patient plasmas and sera prepared from patient plasmas using a FDP-exclusive blood collecting tube were measured by using the anti-FDP monoclonal antibody-sensitized latex reagent (the reagent of the present method) produced in 3-b) as mentioned above and an anti-FDP polyclonal antibody-sensitized latex reagent (trade name: "FDPL Test", available from Teikoku Hormone MFG Co., Ltd.), and compared to each other. On a slide plate, 30 µl of a diluted specimen, 30 µl of a buffer solution and 35 µl of a reagent for slide were mixed, and presence or absence of agglutination after 2 minutes rocking was judged and a FDP concentration (µg/ml) was measured from the diluted amount of the specimen. The results are shown in Table 2. From Table 2, the plasma-measured values according to the present method accorded with the sera-measured values according to the conventional method.

**Table 2**

| | FDP concentration (µg/ml) | |
|---|---|---|
| Specimen | Reagent of the present invention (plasma) | Conventional reagent (plasma) |
| 1 | 2.5 | 2.5 |
| 2 | 5 | 5 |
| 3 | 10 | 10 |
| 4 | 0.5 | 0.5 |
| 5 | 30 | 30 |
| 6 | 50 | 55 |
| 7 | 20 | 20 |

### Utilizability in industry

The monoclonal antibody of the present invention is capable of measuring FDP by using plasma as a specimen according to the immunological agglutination method without using serum in which fibrinogen is removed, and further, a method of measuring FDP using the monoclonal antibody can be provided.

## Claims

1. A monoclonal antibody having properties of specifically reacting with X fraction and Y fraction, but not reacting with D fraction, E fraction, DD fraction and human fibrinogen, in human fibrin/fibrinogen degradation products by plasmin.

2. A monoclonal antibody having properties of reacting with X fraction, Y fraction, D fraction, E fraction and DD fraction, but not reacting with human fibrinogen, in human fibrin/fibrinogen degradation products by plasmin.

3. In a method of measuring human fibrin/fibrinogen degradation products by plasmin in plasma according to an immunological agglutination method, the measurement method comprises using, as antibodies, two kinds of monoclonal antibodies of
(1) a monoclonal antibody having properties of specifically reacting with X fraction and Y fraction, but not reacting with D fraction, E fraction, DD fraction and human fibrinogen; and
(2) a monoclonal antibody having properties of reacting with X fraction, Y fraction, D fraction, E fraction and DD fraction, but not reacting with human fibrinogen, in human fibrin/fibrinogen degradation products by plasmin.

4. The measurement method according to Claim 3, wherein the measurement method according to the immunological agglutination method is a method in which the degree of agglutination is measured by using a latex reagent in which the monoclonal antibody is sensitized to latex particles.

5. The measurement method according to Claim 4, wherein the degree of agglutination is measured spectroscopic manner.

6. The measurement method according to Claim 4, wherein the degree of agglutination is measured with eyes.
